# EUROPEAN PATENT APPLICATION

(11) **EP 3 219 279 A1**
(43) Date of publication of application: **20.09.2017**
(21) Application number: 17161245.0
(22) Date of filing: 16.03.2017
(51) Int. Cl.: A61B 18/14, A61B 18/00

(54) **SKIN TREATMENT APPARATUS**

(30) Priority: 17.03.2016 US 201615072409
(71) Applicant: Syneron Medical Ltd., 2069200 Yoqneam Illit (IL); Candela Corporation, Wayland, MA 01778 (US)
(72) Inventor: Eckhouse, Shimon, 34980 HAIFA (IL); Schomacker, Kevin T., MAYNARD, MA 01754 (US)
(74) Representative: ZBM Patents - Zea, Barlocci & Markvardsen

(57) **Abstract**

The application discloses a number of apparatuses suitable for selective skin treatment. In one example, a pair of RF electrodes is applied to skin. The RF electrodes receive RF energy from an RF energy generator. The RF electrodes are also configured to apply positive pressure to a segment of skin located between the pair of RF electrodes to partially or completely occlude the blood flow in one or more vessels in the segment of skin located between the pair of RF electrodes. Occlusion of the blood flow reduces heat dissipation by the skin segment located between the pair of RF electrodes and supports further increase of temperature of both the skin segment and vessels located between the pair of electrodes.

## Description

### TECHNOLOGY FIELD

The present apparatus and method are related to methods and apparatuses for treating skin and in particular to skin rejuvenation and tightening procedures that reduce the appearance of abnormal skin vessels and wrinkles that occurs in aged and photoaged skin.

### BACKGROUND

Radiofrequency (RF) energy treatment is a technology widely used for many cosmetic, aesthetic and medical skin treatments. Radio Frequency (RF) energy is applied or coupled to skin by at least one electrode and frequently by two or more electrodes. Application of RF energy to skin heats the segment of skin or tissue located between the electrodes. Such skin heat treatment is a popular non-invasive treatment method for skin rejuvenation, skin tightening, vessel shrinkage, wrinkles reduction, collagen rejuvenation, acne treatment and other skin treatment procedures.

Most of the skin treatments are successful only when the skin reaches an optimal treatment temperature. In reaching the optimal treatment temperature, several factors are considered, such as location of the skin segment, energy coupled to the skin, electrode size and orientation of the electrode relative to the blood vessels.

The following patents US 7,041,100 and US 7,238,183 both to Kreindel and assigned to the assignee of the present patent application may be of interest.

### DEFINITIONS

Skin rejuvenation is a general term for skin treatment that includes removal of vascular and pigmented lesions, improving skin texture, and reduction of fine lines and wrinkles.

In the context of the present disclosure the terms "skin" and "tissue" are used interchangeably and have the same meaning. The terms include the outer skin layers such as stratum corneum, epidermis, dermis, and the deeper subcutaneous layers such as adipose tissue.

The term "bipolar electrodes" as used in the present disclosure means that the RF induced current flow passes between two usually identical electrodes each placed on the skin surface a short distance apart from each other. RF energy is applied to the volume of skin/tissue to be treated and the propagation of the current is limited predominantly to the volume of skin/tissue between the electrodes.

The term "skin cooling temperature" as used in the present disclosure means a temperature of 30 degrees C or less, when starting at 32 degrees C.

The term "non-therapeutic treatment temperature" as used in the present disclosure means a treatment temperature of 37 degrees C or less, when starting at 32 degrees C.

The term "therapeutic treatment temperature" as used in the present disclosure means a treatment temperature of 45 degrees C or more, when starting at 32 degrees C.

### SUMMARY

A number of methods and apparatuses suitable for selective skin treatment have been described. In one example, a pair of RF electrodes is applied to skin. The RF electrodes receive RF energy from an RF energy generator and couple the RF energy to the segment of skin located between the pair of RF electrodes. The RF electrodes are also configured to apply positive pressure to a segment of skin located between the pair of RF electrodes to partially or completely occlude the blood flow in one or more vessels in the segment of skin located between the pair of RF electrodes. Occlusion of the blood flow in a segment of skin located between the pair of RF electrodes reduces heat dissipation by the skin segment located between the pair of RF electrodes and supports further increase of temperature of both the skin segment and vessels located between the pair of electrodes.

Described is also an applicator for selective skin segment temperature increase. The applicator includes a pair of RF electrodes configured to apply RF energy to a segment of skin to raise skin temperature to a therapeutic treatment temperature. The pair of RF electrodes is further configured to apply pressure to skin and at least partially occlude blood flow in a segment of skin located between the pair of RF electrodes to reduce heat dissipation by the skin segment located between the pair of electrodes.

According to another example, for selective heating of a skin segment, a pair of RF electrodes is applied to the segment of skin. The RF electrodes are configured to couple RF energy to the segment of skin such as to raise the skin temperature to a first nontherapeutic treatment temperature. A cryogenic fluid is sprayed onto the segment of skin for cooling the superficial layers of skin. Continued application of RF energy to the RF electrodes further raise temperature preferentially of deeper skin layers located between the pair of RF electrodes to a second therapeutic treatment temperature.

According to another example, for selective heating of a skin segment, a pair of RF electrodes is applied to the segment of skin. A cryogenic fluid is sprayed onto the segment of skin for cooling the superficial layers of skin to a first skin cooling temperature. The RF electrodes are configured to couple RF energy to the segment of skin such as to raise the skin temperature of the superficial layers of the segment of skin to a nontherapeutic treatment temperature and of the deeper layers of the segment of skin to a therapeutic treatment temperature.

According to another example, for selective heating of a skin segment, a pair of RF electrodes is applied to the segment of skin. A cryogenic fluid is sprayed onto the segment of skin for cooling the superficial layers of skin to a first skin cooling temperature. The RF electrodes are configured to couple RF energy to the segment of skin such as to raise the skin temperature of the segment of skin to a nontherapeutic treatment temperature and of blood vessels in the segment of skin to a therapeutic treatment temperature.

According to an additional example, the method of selective skin segment temperature increase includes application to a segment of skin of a pair of RF electrodes configured to couple RF energy to the segment of skin to raise skin temperature to a first nontherapeutic treatment temperature. The RF electrodes are also configured to apply positive pressure to a segment of skin located between the pair of RF electrodes and at least partially occlude blood flow in a segment of skin located between the pair of RF electrodes. The method further includes use of a cryogenic fluid for cooling the superficial layers of skin; and continue application of RF energy to the electrodes to further increase temperature to a second therapeutic treatment temperature of selected skin segment layers located deeper between the pair of electrodes.

The applicator for performing this treatment includes a pair of RF electrodes configured to apply RF energy to a segment of skin to raise skin temperature to a first skin treatment temperature and a cryogenic cooling fluid delivery unit configured to direct a spray of cryogenic vapors to cool skin located between the RF electrodes. The pair of RF electrodes is further configured to apply pressure to skin and at least partially occlude blood flow in a segment of skin located between the pair of RF electrodes to reduce heat dissipation by the skin segment located between the pair of electrodes.

### BRIEF LIST OF DRAWINGS AND THEIR DESCRIPTION

FIG. 1 is an example of the relative increase in temperature of blood when compared to surrounding dermal tissue for a 0.1 second bipolar RF pulse;
FIG. 2 is an example of dielectric loss (higher losses denote better ohmic heating) for blood and dermal tissue as a function of RF energy frequency;
FIG. 3 illustrates the ratio of dielectric loss for blood relative to dermal tissue as a function of RF energy frequency;
FIG. 4 is a block diagram of an apparatus for conducting present skin treatment according to an example;
Fig. 5 is an example of an applicator containing a pair of RF electrodes for conducting present skin treatment;
Fig. 6 is an additional example of an applicator containing a pair of RF electrodes for conducting present skin treatment;
FIG. 7 is an example of an applicator configured to provide a combination of cryogenic skin surface cooling with bi-polar RF energy application; and
Fig. 8 is an example of graphs illustrating the reduction in electrical conductivity in the more superficial skin layers as the result of skin surface cooling by cryogenic spray, the net result leading to more RF heating of the deeper skin layers.

### DESCRIPTION

One of the obstacles of radiofrequency skin heat treatments are nonuniform heating in the treated skin segment and difficulty in reaching desired skin temperature, since blood flow acts as a heat sink and distributes the heat over relatively large skin areas. The present disclosure suggests application of positive pressure to a treated by RF energy skin segment to reduce or even partially (temporarily) occlude the blood flow and associated with it heat loss or heat dissipation by the skin segment located between the pair of electrodes.

The present disclosure makes use of increase in blood conductivity with temperature to enhance RF energy selectivity towards blood vessels as well as optimize the RF energy frequency and application for skin rejuvenation and blood vessels treatment.

Disclosed is also a method of pushing RF heating and increase of temperature of deeper tissue layers by cooling the skin/tissue surface with cryogenic fluids. The cooling reduces the electrical conductivity of superficial skin layers or epidermal and upper dermal surface layers. The depth of the heating achieved is dependent on the relations between the amount and time of cooling fluid application and RF energy application.

In one example, bipolar RF power/energy is applied between a pair (or pairs) of adjacent RF electrodes, leading to increase of the skin segment temperature. Concurrently, a certain positive pressure is applied to the skin located between the electrodes to reduce or even partially occlude the blood perfusion between the two electrodes in the skin segment and reduce heat dissipation by containing a reduced amount of the heated blood to the region between the two electrodes. Application of RF energy to the skin segment continues to further increase temperature to a therapeutic treatment temperature.

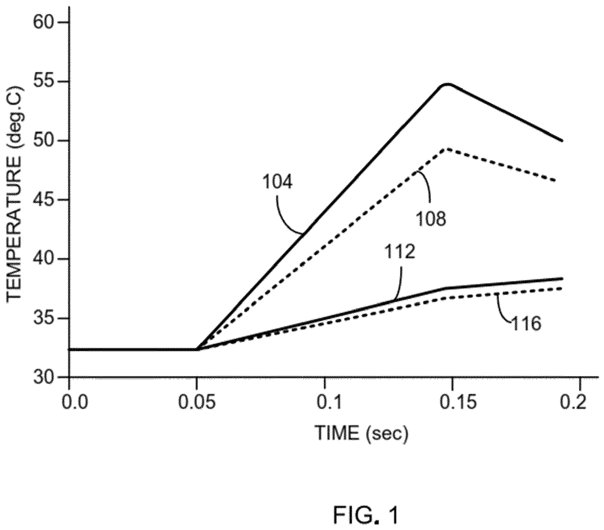

FIG. 1 is an example of relative increase in temperature of blood when compared to surrounding dermal tissue for a 0.1 second bipolar RF pulse at a frequency of 1 MHz. The increase in conductivity (σ) of blood and surrounding dermal tissue occurs when heat is applied to tissue σ = σₒ(1+α(T-Tₒ)), where α is typically between 1 %/K to 3%/K for biological tissue, and temperature is given on the Kelvin scale.

For temperature independent conductivity, σ = σₒ (dashed lines), reference numeral 108 (FIG. 1) shows more heating for the 0.5mm blood vessel when compared to surrounding dermis 116, simply because the conductivity of blood is nearly 4-fold larger than the conductivity of dermis. When including the temperature dependent term (solid lines), reference numeral 104 shows even more heating of a 0.5mm blood vessel when compared to surrounding skin tissue 112 due to the changes in conductivity with temperature. Coupling of RF energy to skin leads to an increase of the skin temperature of 5 degrees C, for example to a first non-therapeutic treatment temperature of 37 degrees C, when starting for example, at 32 degrees C.

Coupling of RF energy to blood leads to an even faster, increase in blood conductivity and temperature increase and supports faster achievement of a second therapeutic treatment temperature of 53 degrees C when starting at 32 degrees C.

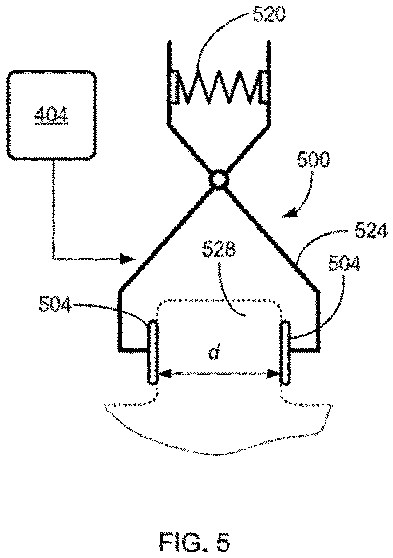

The bipolar RF electrodes (504 FIG. 5) could be one to two centimeter wide and located at a distance of 0.5 cm to 1.5 cm apart of each other. The bi-polar RF electrodes 504 could be placed in different orientations with respect to the blood vessels in the treated skin segment, although faster temperature increase has been achieved, when the RF electrodes were placed such that RF induced current flow is parallel with blood vessel direction. With such an orientation, a larger fraction of the RF current is directly through the blood vessel.

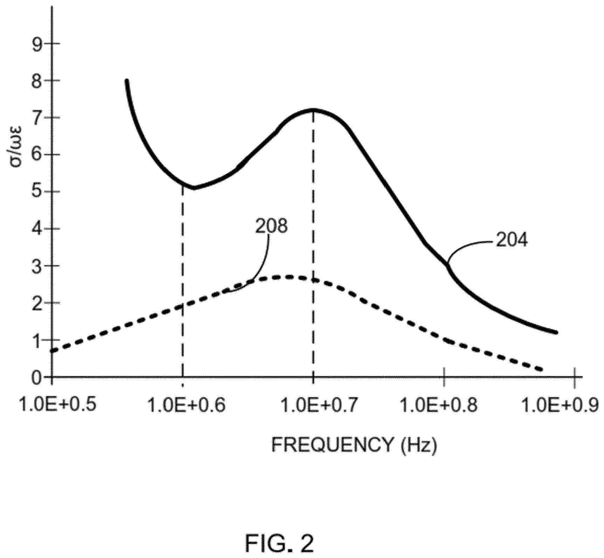

In addition to the dependence on temperature, the electrical conductivity of tissue depends on the frequency of RF energy that induces the current flow in the tissue. FIG. 2 is an example of the dielectric loss (higher losses denote better ohmic heating) for blood and dermal tissue as a function of RF energy frequency. The dielectric loss is the ratio of the lossy electrical conductivity to the lossless dielectric component. The larger the ratio, the more tissue is heated via ohmic current. Curve 204 relates to the dielectric loss for blood and broken line 208 illustrates the dielectric loss for dermal tissue under the same conditions.

**Table 1 below shows skin/tissue conductivity of different skin segments.**

| **Skin Segment** | **Electric Conductivity (S/m) At 1MHz** |
|---|---|
| Blood | 0.82 |
| Bone | 0.024 |
| Fat | 0.035 |
| Dry skin | 0.013 |
| Wet skin | 0.22 |

| | |
|---|---|
| http://niremf.ifac.cnr.it/tissprop/htmlclie/htmlclie.php. Data from C.Gabriel and colleagues at the Brooks Air Force Base, U.S.A. | |

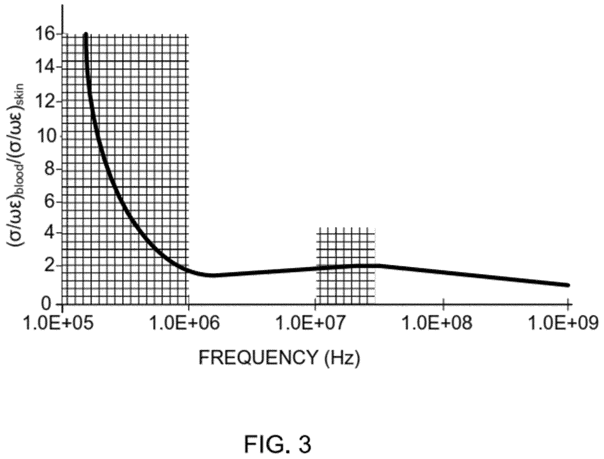

FIG. 3 illustrates the ratio of the dielectric loss for blood relative to dermal tissue as a function of RF energy frequency applied to the skin. The larger the ratio, the more selective the RF energy is to heating blood compared to heating surrounding dermal tissue. The graph indicates that at a frequency of RF energy of about 0.2 MHz, selective heating of blood is about 4 times higher than at RF energy frequency of 1.0 MHz.

FIG. 3 also indicates that use of RF frequencies around 10MHz is more efficient at heating blood compared to 1.0 MHz used for heating the dermis. Use of RF frequencies between 0.1 and 1 MHz and around 10 MHz to promote significant selectivity towards vessel heating could help achieve therapeutic blood vessel temperatures while reduce potential overheating of the dermis.

As demonstrated above, blood and parts of the body with high blood content have the highest electrical conductivity. The pattern of distribution of electrical conductivity demonstrates the possibility for selective treatment of blood vessels using RF energy of proper frequency.

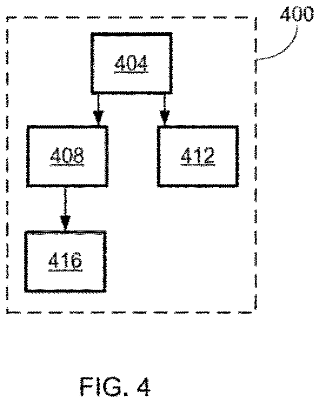

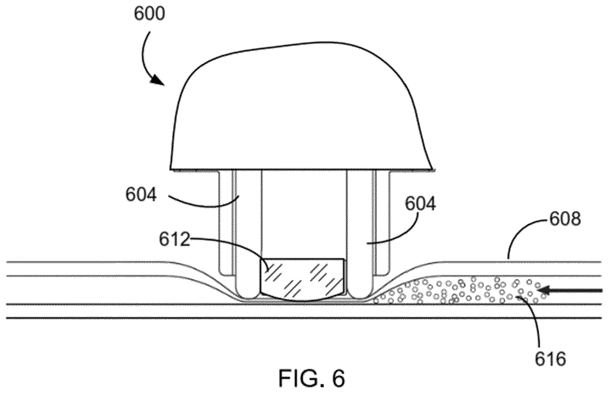

FIG. 4 is a block diagram of an apparatus for conducting present skin treatment according to an example. Apparatus 400 includes a personal computer (PC) 404, an RF energy generator or RF energy supply unit 408 including a module configured to control supply of RF energy to an applicator 416 and more specifically to applicator 416 RF electrodes. The control includes the amplitude and application time of the RF energy. Apparatus 400 also includes a skin cryogenic cooling unit 412 configured to cool at least the epidermal and superficial dermal or skin layers of the segment of skin. Skin cooling unit 412 could be such as a cryogenic spray cooling device configured to direct a spray of cryogenic vapors to cool skin fold 528 (FIG. 5) located between the RF electrodes 504 of applicator 416. Skin cooling unit 412 could be a refrigerator unit designed to supply a coolant via a pump to the pair of RF electrodes 604 (FIG. 6) and indirectly to a part placed between the electrode 612 (FIG. 6). The refrigerator unit could be a compact compressor or thermoelectric cooling module. In addition, the thermoelectric module could be mounted directly onto the pair of bipolar RF electrodes.

RF energy generator 408 and skin cooling unit 412 could be located in the same packaging as the control computer or could have separate packaging. In some examples, RF energy generator 408 and skin cooling unit 412 could be located in applicator 500 (FIG. 5).

After achieving the first skin cooling temperature, control computer 404 could start application of RF energy to the skin to reach a first non-therapeutic temperature in skin and achieve a second therapeutic treatment temperature in the target of interest. The target of interest can be an underlying blood vessel or the deeper skin layers.

Fig. 5 is an example of an applicator including a pair of RF electrodes 504 for conducting present selective skin treatment. The distance d between the pair of RF electrodes 504 is variable and the distance d determines thickness of a skin fold 528 formed and located between the pair of RF electrodes 504. RF electrodes 504 could be mounted on a spring 520 activated mount 524. Alternatively, RF electrodes 504 could be mounted on a calibrated ratchet type mount having a predetermined number of positions. In order to form a skin fold 528 RF electrodes 504 (or spring 520 and/or calibrated ratchet type mount) are configured to apply to skin located between the RF electrodes a mechanical force defining a positive pressure of 0.1 Mp to 2.0 Mp. Application of positive pressure to a treated by RF energy skin segment occludes and reduces partially or completely (temporarily) the blood flow (blood perfusion) between the two RF electrodes and associated with it heat dissipation.

An electrical contact improving gel could be used between electrodes 504 and skin fold 528 to avoid formation of air pockets or to wet the outer dry layer of skin thereby reducing the electrical impedance. In some examples electrodes 504 could be made of a porous and electrically conductive material. Such electrode structure could facilitate removal of air trapped between the skin fold 528 and the RF electrode.

Skin cooling unit 412 (FIG. 4) is configured to couple skin coolant to a segment of skin to maintain a segment of skin to a first skin cooling temperature. RF energy supply unit 404 (FIG. 4) controlled by control computer 404 is configured to then supply RF energy to the RF electrodes 504. For example, after achieving the first skin treatment temperature, control computer 404 could start application of RF energy to the skin to reach the second therapeutic skin treatment temperature.

Fig. 6 is an additional example of an applicator containing a pair of RF electrodes for conducting present skin treatment. Applicator 600 includes a pair of electrodes 604 supporting application of positive (downward) pressure to the segment of skin 608 located between the electrodes 604. Numeral 612 marks a part configured to support application of additional pressure to the skin surface and to occlude blood vessels 616 located beneath the skin surface. Occlusion of blood vessels reduces the amount of blood and reduce the heat dissipation by the skin segment located between the pair of electrodes. In one example, part 612 could be made from transparent material and support application of additional type of energy, for example light energy. In another example, part 612 could be made with a material having good thermal conductivity chilled via the cooled pair of bipolar RF electrodes, thereby cooling the skin surface.

A combination of cryogenic skin surface cooling with bi-polar RF energy application also supports heating and increase of temperature of deeper skin layers, than currently existing RF electrode cooling methods. At present cooling is predominantly used to minimize skin burns from hot spots that occur at the RF electrodes. Cryogenic skin surface cooling reduces skin surface temperature and increases resistivity of the upper layers of the skin. The increased resistivity (or reduced conductivity) drives RF induced current flow into skin layers with higher conductivity and namely into the deeper uncooled skin layers to further increase temperature of the deeper skin layers.

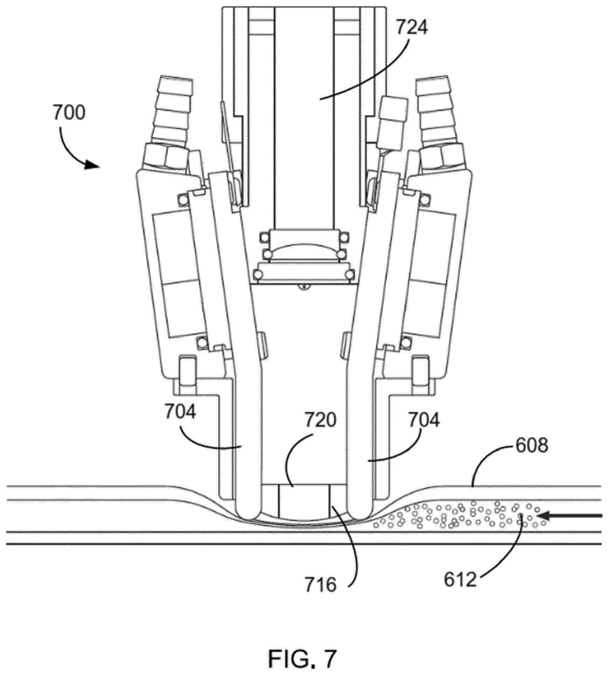

The aim of these techniques is to heat dermis and subcutaneous tissue while minimizing heating of the epidermis and upper dermis, and consequently reducing damage to the superficial skin or dermal layers FIG. 7 is an example of an applicator configured to provide a combination of cryogenic skin surface cooling with bi-polar RF energy application. Applicator 700 includes at least a pair of RF electrodes 704 configured to apply positive (downward) pressure to the segment of skin 608 located between RF electrodes 704 and squeeze out blood from blood vessel/s 612 located beneath the RF electrodes. This reduces the amount of blood between the RF electrodes and a ring type part 716 with opening 720 could be used to support cryogenic spray-to-skin access and increase the surface pressure. Cryogenic spraying delivery unit 724 is also included in applicator 700, although cryogenic fluid could be conducted through a flexible isolated tubing. RF electrodes 704 could be cooled by a separate cooling device (not shown) that could provide a cooling fluid such as water or other cooling fluid.

The bipolar RF energy can be delivered as a single pulse or as multiple RF energy pulses. Cryogenic spray can be delivered as a single burst prior to or at the beginning of the RF energy pulse, or in multiple cryogenic spray bursts at various times during, and after the RF pulse. For example, a complete treatment pulse could include four DCD/RF sub-pulses delivered within one or two seconds, preferably less than 1 second. The duration of each DCD sub-pulse could be varied from 10 to 100ms and preferably could be 10 or 20ms in duration. The duration of the each RF sub-pulse could be varied from 50 to 200ms, with a preferable on time of 100ms. Using a 200W RF generator this corresponds to a sub-pulse RF energy from 10 to 40J, for 4 sub-pulses a total RF energy between 40 and 160J. By selecting a proper combination of RF energy delivery time and intensity and cryogenic spray delivery, it is possible to deliver RF heating deeper into tissue without overcooling the skin surface. This becomes possible, since surface cooling has reduced electrical conductivity of the upper skin layers and supply of RF energy created a current flow through the hotter deeper located skin layers. In addition, by splitting the RF pulse into smaller RF sub-pulses, more RF energy can be delivered to tissue while avoiding skin surface burns from electrode hotspots.

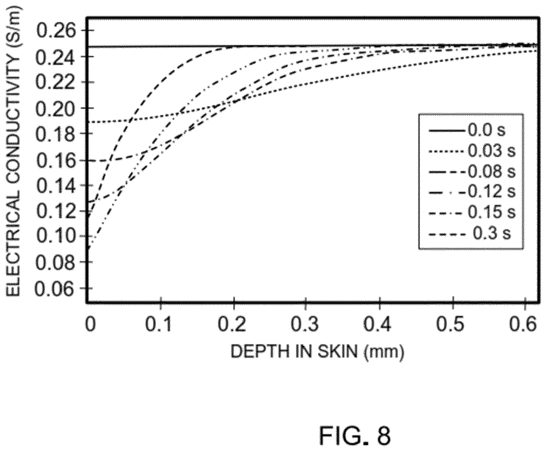

FIG. 8 illustrates the electrical conductivity of skin at various time and depths into tissue during and after a single 50ms cryogenic spray is delivered to the skin surface. RF energy delivery and heating is dependent on electrical conductivity so will lead to more heating and rise of temperature of the deeper skin layers that have the higher electrical conductivity. The increase in electric conductivity with RF heating combined with reduced electrical conductivity at the surface due to surface cooling helps to drive RF heating deeper into tissue away from tissue surface.

The present document discloses a method and apparatus for RF energy delivery to deeper skin layers and selective heating of the desired skin layers. This is achieved by increasing the conductivity of the skin with increase in skin temperature, proper selection of the RF energy frequencies and application of positive pressure between the electrodes to at least partially occlude the blood flow and reduce blood perfusion in the skin segment and associated with it heat dissipation.

The document also discloses a method of RF energy delivery to deeper skin layers by selecting a proper RF energy pulse duration and cryogenic skin surface cooling.

While the method and apparatus have been described with respect to a number of examples, it will be appreciated that many other variations, modifications and applications of the method and apparatus may be made.

For reasons of completeness, various aspects of the invention are set out in the following numbered clauses:
Clause 1. A method for selective skin segment temperature increase comprising:
   applying to a segment of skin a pair of RF electrodes;
   applying a cryogenic fluid for cooling the superficial skin layers to a first skin cooling temperature;
      the method characterized in that
   applying positive pressure to the segment of skin (608) located between the pair of RF electrodes (504, 604) to at least partially occlude blood flow and reduce heat dissipation in a segment of skin (608) located between the pair of RF electrodes (504, 604); and
   coupling RF energy to the segment of skin (608) to raise temperature of deeper layers of skin to a second therapeutic skin treatment temperature. Clause 2. The method according to clause 1 wherein application of positive pressure to a segment of skin (608) located between the pair of RF electrodes (504, 604) is by varying distance between the pair of electrodes (504, 604).
Clause 3. The method according to any of clauses 1 or 2, wherein continue application of RF energy to the RF electrodes (504, 604) causes faster increase temperature of the skin segment, where blood flow is occluded. Clause 4. The method according to any of clauses 1 to 3, wherein applying a pair of RF electrodes (504, 604) to a segment of skin between the electrodes (504, 604) includes orienting the electrodes (504, 604) such that RF energy induced current flow is parallel with blood vessel direction. Clause 5. The method according to any of clauses 1 to 4, wherein applying positive pressure includes application of a mechanical force on the segment of skin.
Clause 6. The method of clause 5, wherein employing a mechanical force includes using the pair of RF electrodes (504, 604) to apply the mechanical force.
Clause 7. The method of clause 5, wherein employing a mechanical force includes using a spring (520) to apply the mechanical force.
Clause 8. The method of any of clauses 1 to 7, wherein the positive pressure applied is 0.1 Mp to 2.0 Mp.
Clause 9. An apparatus for selective skin segment temperature increase comprising:
   a RF energy generator (408);
   a pair of RF electrodes (504, 604) configured to apply RF energy to a segment of skin (608);
   a cryogenic cooling fluid delivery unit (412) configured to cool at least the superficial skin layers of the segment of skin (608) between the RF electrodes to a first non-therapeutic temperature; and
   a PC configured to control supply of RF energy to the RF electrodes (504, 604);
   characterized in that the pair of electrodes (504, 604) is further configured to apply positive pressure to a segment of skin and at least partially occlude blood flow in a segment of skin (608) located between the pair of RF electrodes (504, 604) to reduce heat dissipation by the skin segment (608) located between the pair of electrodes (504, 604).
Clause 10. The apparatus according to clause 9, wherein distance between the pair of RF electrodes (504, 604) configured to couple RF energy to a segment of skin is a variable distance.
Clause 11. The apparatus according to any of clauses 9 or 10, wherein distance between the pair of RF electrodes (504, 604) configured to couple RF energy determines thickness of a skin fold (528) located between the pair of electrodes (504, 604).
Clause 12. The apparatus according to any of clauses 9 to 11, wherein in order to form a skin fold (528) the RF electrodes (504, 604) apply to skin a positive pressure of 0.1 Mp to 2.0 Mp.
Clause 13. The apparatus according to any of clauses 9 to 12, wherein a spring (520) applies positive pressure to a skin fold (528) located between the pair of electrodes (504, 604).
Clause 14. The apparatus according to any of clauses 9 to 13 wherein the RF electrodes are mounted on a calibrated ratchet type mount.
Clause 15. The apparatus according to any of clauses 9 to 14 further comprising a cryogenic skin cooling device (412) configured to direct a spray of cryogenic vapors to cool skin fold (528) located between the RF electrodes (504, 604).
Clause 16. The apparatus according to any of clauses 9 to 15 wherein the RF electrodes (504, 604) are made of a porous and electrically conductive material to facilitate removal of air trapped between a skin fold (528) and RF electrode (504, 604).
Clause 17. The apparatus according to any of clauses 9 to 16 wherein multiple RF energy and DCD sub-pulses are delivered to a segment of skin (608) between a pair of bipolar RF electrodes (504, 604).
Clause 18. The apparatus according to clause 17 wherein the DCD sub-pulses are 10ms to 100ms long in duration.
Clause 19. The apparatus according to any of clauses 17 or 18 wherein RF energy sub-pulses are 50ms to 200ms long in duration.
Clause 20. An applicator for selective skin segment temperature increase comprising:
   a pair of RF electrodes (504, 604) configured to apply RF energy to a segment of skin (608) to raise skin temperature to a first skin treatment temperature; and
   wherein the pair of electrodes (504, 604) is further configured to apply pressure to a segment of skin (608) and at least partially occlude blood flow in a segment of skin (608) located between the pair of RF electrodes (504, 604) to reduce heat dissipation by the segment of skin (608) located between the pair of electrodes (504, 604).
Clause 21. The method according to any of clauses 1 to 8 wherein employing an RF electrode (504) made of a porous and electrically conductive material to facilitate removal of air trapped between skin fold (528) and the RF electrode (504).

## Claims

1. An apparatus for selective skin segment temperature increase comprising:
a RF energy generator (408);
a pair of RF electrodes (504, 604) configured to apply RF energy to a segment of skin (608);
a cryogenic cooling fluid delivery unit (412) configured to cool at least the superficial skin layers of the segment of skin (608) between the RF electrodes to a first non-therapeutic temperature; and
a PC configured to control supply of RF energy to the RF electrodes (504, 604);
**characterized in that** the pair of RF electrodes (504, 604) is further configured to apply positive pressure to a segment of skin and at least partially occlude blood flow in a segment of skin (608) located between the pair of RF electrodes (504, 604) to reduce heat dissipation by the skin segment (608) located between the pair of electrodes (504, 604).

2. The apparatus according to claim 1, wherein distance between the pair of RF electrodes (504, 604) configured to couple RF energy to a segment of skin is a variable distance.

3. The apparatus according to any of claims 1 or 2, wherein distance between the pair of RF electrodes (504, 604) configured to couple RF energy determines thickness of a skin fold (528) located between the pair of electrodes (504, 604).

4. The apparatus according to any of claims 1 to 3, wherein in order to form a skin fold (528) the RF electrodes (504, 604) apply to skin a positive pressure of 0.1 Mp to 2.0 Mp.

5. The apparatus according to any of claims 1 to 4, wherein a spring (520) applies positive pressure to a skin fold (528) located between the pair of RF electrodes (504, 604).

6. The apparatus according to any of claims 1 to 5, wherein the pair of RF electrodes are mounted on a calibrated ratchet type mount.

7. The apparatus according to any of claims 1 to 6, further comprising a cryogenic skin cooling device (412) configured to direct a spray of cryogenic vapors to cool skin fold (528) located between the RF electrodes (504, 604).

8. The apparatus according to any of claims 1 to 7, wherein the pair of RF electrodes (504, 604) are made of a porous and electrically conductive material to facilitate removal of air trapped between a skin fold (528) and RF electrode (504, 604).

9. The apparatus according to any of claims 1 to 8, wherein multiple RF energy and DCD sub-pulses are delivered to a segment of skin (608) between a pair of bipolar RF electrodes (504, 604).

10. The apparatus according to claim 9, wherein the DCD sub-pulses are 10ms to 100ms long in duration.

11. The apparatus according to any of claim 9 or 10, wherein RF energy sub-pulses are 50ms to 200ms long in duration.
